# EUROPÄISCHE PATENTSCHRIFT

(11) **EP 0 585 570 B1**
(45) Veröffentlichungstag und Bekanntmachung des Hinweises auf die Patenterteilung: **18.11.1998**
(21) Anmeldenummer: 93111172.8
(22) Anmeldetag: 13.07.1993
(51) Int. Cl.: C12N 15/13, C12N 15/62, C12P 21/08, A61K 39/395, C07K 16/18

(54) **Granulozytenbindende Antikörperkonstrukte, ihre Herstellung und Verwendung**
Antigranulocyte antibody construct, preparation and use thereof
Greffon d'anticorps anti-granulocytes, sa préparation et usage

(30) Priorität: 05.08.1992 DE 4225853
(43) Veröffentlichungstag der Anmeldung: 09.03.1994
(73) Patentinhaber: HOECHST AKTIENGESELLSCHAFT, 65929 Frankfurt am Main (DE)
(72) Erfinder: Seemann, Gerhard, D-35041 Marburg-Elnhausen (DE); Bosslet, Klaus, D-35037 Marburg (DE)
(74) Vertreter: Fischer, Hans-Jürgen, Dr.

(56) Entgegenhaltungen:
- EP-A- 0 328 404
- EP-A- 0 388 914
- EP-A- 0 501 215
- WO-A-91/09968
- WO-A-92/01059

## Beschreibung

Die vorliegende Erfindung beschreibt granulozytenbindende Antikörperkonstrukte, die von dem monoklonalen Mausantikörper MAk BW 250/183 (EP-A1-0 388 914) abgeleitet wurden, sowie ein Verfahren zu ihrer Herstellung und ihre Verwendung.

Der monoklonale Antikörper MAk BW 250/183 ist ein monoklonaler Mausantikörper des IgG1-Kappa Typs, der gegen das "nonspecific crossreacting antigen" (NCA) 95 und gegen das carcinoembryonale Antigen (CEA) gerichtet ist und keine unerwünschten Kreuzreaktionen mit menschlichen Normalgeweben außer der Colonmucosa hat. Seine Bindung an Granulozyten und Granulozytenvorläufer im Knochenmark ermöglicht auch eine therapeutische Anwendung.

Bei der therapeutischen Anwendung von monoklonalen Antikörpern (MAk) bei Menschen kommen im allgemeinen Antikörper zum Einsatz, die aus Nagern, vor allem aus Mäusen isoliert wurden. Die wiederholte Applikation hoher Dosen von Antikörpern nicht menschlichen (xenogenen) Ursprungs kann jedoch zu einer Immunreaktion der Patienten führen. Sie entwickeln nach etwa 10 - 14 Tagen anti-Maus Immunglobulin-Antikörper (HAMA). Eine solche Therapie muß deshalb nach 10 Tagen abgebrochen werden und kann nicht wieder aufgenommen werden.

Mit Hilfe molekularbiologischer Methoden ist es mittlerweile gelungen, die xenogenen Antikörper so zu verändern, daß sie für den Menschen nicht mehr oder nur noch sehr schwach immunogen sind und trotzdem ihre Antigenbindungseigenschaften behalten. In einem ersten Schritt wurden dabei in den Genen, die für die H- und L-Ketten der xenogenen MAk kodieren, die konstanten Exons (C_{H}1,H, C_{H}2, C_{H}3, C_{L}) gegen Exons aus menschlichen Immunglobulingenen ausgetauscht (Boulianne, G.L., Nobumichi Hozumi, and Marc J. Shulman (1984) Nature 321, 643-646). Auf diese Weise erhält man sogenannte chimäre Antikörper, die aus variablen Domänen xenogenen Ursprungs und menschlichen konstanten Bereichen bestehen. Diese chimären Antikörper enthalten noch etwa 30 % xenogenen Proteinsequenzen, so daß bei ihrer Verwendung in der Immuntherapie noch immer eine, wenn auch schwächere, Immunreaktion der Patienten zu erwarten ist (Brüggemann, M., Greg Winter, Herman Waldmann, and Michael S. Neuberger (1989), J. Exp. Med. 170, 2153-2157).

Um die Immunogenität von xenogenen Antikörpern weiter zu reduzieren, wurde von G. Winter und M. Neuberger1986 eine Technik entwickelt, bei der nur die CDR-Loops der V_{L}- und V_{H}-Domänen der xenogenen Antikörper auf V_{L}- und V_{H}-Domänen von menschlichen Antikörpern übertragen werden (Jones, P.T., Paul H. Dear, Jefferson Foote, Michael S. Neuberger, and Greg Winter (1986) Nature 321, 522-525), ein Vorgang der als "Humanisierung" bezeichnet wird und sich auf der Ebene der V_{H}- und V_{L}-Gene abspielt. Die strukturelle Variabilität der V_{H}- und V_{L}-Domänen beschränkt sich im allgemeinen auf die CDR-Loops ["CDR" steht für Complementarity Determining Regions].

In EP 0 501 215 wird ein Fusionsprotein für die Prodrug-Aktivierung der Formel huTuMAK-L-β-Gluc beschrieben, wobei huTuMAK für einen humanisierten oder humanen tumorspezifischen monoklonalen Antikörper steht, L Linker bedeutet und β-Gluc für humane β-Glucuronidase steht.

In WO 92/01059 wird ein humanisierter Antikörper beschrieben, der spezifisch für ein carcinoembryonales Antigen ist und der einen Antigenbindebereich aufweist, der aus wenigstens einer komplementären Bestimmungsregion besteht, die sich von der variablen Domäne des monoklonalen Maus Antikörpers A5B7 ableitet und der Rest des humanisierten Antikörpers von humanen Immunoglobulin abstammt.

In WO 91/09969 werden rekombinante Antikörper beschrieben, dessen Antigenbindebereiche sich von den schweren und/oder leichten Ketten der variablen Regionen eines Anti-CD3-Antikörpers, z.B. OKT 3, ableiten.

In EP 0 328 404 wird die Herstellung von Antikörpern beschrieben, die spezifisch an das Antigen Campath-1 binden, und mindestens eine komplementäre Bestimmungsregion aus der Ratte enthalten und die mit anderen fremden variablen Domäneregionen kombiniert werden können.

In der vorliegenden Patentanmeldung werden nun Antikörperketten beschrieben, welche spezifisch für menschliche Granulozyten sind und bei denen zumindest die CDR-Regionen der schweren und leichten Ketten vom Maus MAk BW 250/183 stammen (EP-A1-0 388 914). Die übrigen Teile von damit hergestellten molekularen Konstrukten, z.B. monoklonalen Antikörpern, stammen vorzugsweise von menschlichen Antikörpern.

Die wesentlichen Schritte bei der Humanisierung eines Antikörpers sind im allgemeinen: die Klonierung und Nukleinsäuresequenzanalyse der spezifischen V_{H}- und V_{L}-Gene, der computerunterstützte Entwurf der synthetischen Oligonukleotide für die Übertragung der CDR-Loops auf die menschlichen V_{H}- und V_{L}-Domänen und die Übertragung der CDR-Loops auf die menschlichen V_{H}- und V_{L}-Domänen mittels spezifischer Mutagenese (Riechman, L., Michael Clark, Herman Waldmann, and Greg Winter (1988) Nature 332, 323-327; Verhoeyen, M., Cesar Milstein, and Greg Winter (1988) Science 239, 1534-1536).

Die Durchführung der Humanisierung des MAk BW 250/183 nach dem von Riechmann und Verhoeyen beschriebenen Verfahren führte nun zu einem humMAk, der eine reduzierte Antigenaffinität im Vergleich zum murinen MAk BW 250/183 hat. Überraschenderweise führte aber der Austausch einer weiteren Aminosäure in der V_{L}-Domäne zu einem humMAk, der eine ähnliche Antigenaffinität wie der Maus Mak BW 250/183 hat.

Gegenstand der Erfindung ist daher eine leichte menschliche granulozytenbindende Antikörperkette, die von einem monoklonalen Mausantikörper abgeleitet wurde, enthaltend ein Polypeptid der Aminosäuresequenz: 
Asp Ile Gln Met Thr Gln Ser Pro Ser Ser Leu Ser Ala Ser
Val Gly Asp Arg Val Thr Ile Thr Cys Arg Ala Ser Ser Ser
Val Ser Tyr Met His Trp Tyr Gln Gln Lys Pro Gly Lys Ala
Pro Lys Pro Leu Ile Tyr Ala Thr Ser Asn Leu Ala Ser Gly
Val Pro Ser Arg Phe Ser Gly Ser Gly Ser Gly Thr Asp Phe
Thr Phe Thr Ile Ser Ser Leu Gln Pro Glu Asp Ile Ala Thr
Tyr Tyr Cys Gln Gln Trp Ser Ser Asn Pro Leu Thr Phe Gly
Gln Gly Thr Lys Val Glu Ile Lys Arg
 oder funktionell äquivalente Varianten davon, die an menschliche Granulozyten oder Granulozytenvorläufer, an NCA 95 und CEA binden, jedoch nicht an menschlichen Normalgewebe außer der Colonmucosa, wobei die Aminosäure in Position 45 (Pro) nicht gegen eine andere Aminosäure ausgetauscht werden darf.

Antikörper, enthaltend mindestens eine leichte menschliche granulozytenbindende Antikörperkette wie oben beschrieben und mindestens eine schwere Antikörperkette, enthaltend ein Polypeptid der Aminosäuresequenz: 
Gln Val Gln Leu Gln Glu Ser Gly Pro Gly Leu Val Arg Pro
Ser Gln Thr Leu Ser Leu Thr Cys Thr Val Ser Gly Phe Ser
Asp Tyr Tyr Met Asn Trp Val Arg Gln Pro Pro Gly Arg Gly
Leu Glu Trp Ile Gly Phe Ile Ser Asn Lys Pro Asn Gly His
Thr Thr Glu Tyr Ser Ala Ser Val Lys Gly Arg Val Thr Met
Leu Arg Asp Thr Ser Lys Asn Gln Phe Ser Leu Arg Leu Ser
Ser Val Thr Ala Ala Asp Thr Ala Val Tyr Tyr Cys Ala Arg
Asp Lys Gly Ile Arg Trp Tyr Phe Asp Val Trp Gly Gln Gly
Ser Leu Val Thr Val Ser Ser
 oder funktionell äquivalente Varianten davon, die an menschliche Granulozyten oder Granulozytenvorläufer, an NCA 95 und CEA binden,
jedoch nicht an menschlichen Normalgewebe außer der Colonmucosa.

Unter funktionell äquivalenten Varianten versteht man im Sinne der Erfindung im allgemeinen solche Varianten der erfindungsgemäßen leichten bzw. schweren Antikörperketten, die an menschliche Granulozyten bzw. Granulozytenvorläufer, an NCA 95 und CEA binden, jedoch nicht an menschlichen Normalgeweben außer der Colonmucosa, wobei bei der leichten Antikörperkette die Aminosäure in Position 45 (Prolin) nicht gegen eine andere Aminosäure ausgetauscht werden darf.

Des weiteren versteht man unter funktionell äquivalenten Varianten im Sinne der Erfindung solche Varianten der erfindungsgemäßen leichten bzw. schweren Antikörperketten, bei denen an Aminosäurepositionen, welche keinen direkten Anteil an der Antigenbindung haben, solche Aminosäureaustausche vorgenommen werden, die nicht zu strukturellen Veränderungen an den Domänenstrukturen und somit auch nicht zur Beeinträchtigung der Antigenbindung führen.

Solche Aminosäurenaustausche können in den CDRs und in den Framework-Regionen der variablen Domänen liegen. Solche Austausche können z.B. konservative Austausche in bezug auf Größe, Polarität und Ladung der Aminosäureseitenkette innerhalb und außerhalb der CDRs sein. Es kann sich z.B. um Austausche zwischen folgenden Aminosäurepaaren handeln, wie zum überwiegenden Teil auch bei Güssow und Seemann beschrieben:

Phe/Tyr; Leu/Met; Val/Ile; Gln/His; Asp/Glu; Arg/Lys;
Trp/Arg; Asn/Asp; Gly/Ala; Ser/Thr; Gln/Asp; Gln/Glu;
Asn/Glu; His/Arg; His/Asn; Ile/Leu; Ile/Met; Leu/Phe;
Leu/Val; Val/Met

Solche Austausche können auch nicht konservative oder beliebige Austausche außerhalb der CDRs sein, welche die Struktur der Domänen und die Faltung der CDRs, und die Interaktionen zwischen V_{H} und V_{L} Domänen nicht beeinträchtigen.

Die Erfindung betrifft weiterhin ein molekulares Konstrukt, enthaltend mindestens eine der erfindungsgemäßen Antikörperketten und mindestens eine weitere mit der Antikörperkette verknüpfte Aminosäurekette, die von der erfindungsgemäßen Antikörperkette verschieden ist.

Ein weiterer Gegenstand der Erfindung ist ein molekulares Konstrukt, bestehend aus der Antikörperkette gemäß Tabelle la oder lc und weitere mit den genannten Sequenzen verknüpfte Aminosäureketten, wobei diese Aminosäureketten verschieden von den erfindungsgemäßen Antikörperketten und vorzugsweise Teile eines humanen Antikörpers sind, beispielsweise Teile der konstanten Bereiche der schweren und leichten Ketten. Spezielle Ausführungsformen solcher molekularer Konstrukte sind beispielsweise ein Single-Domain-Fragment oder Single-Chain-Fragment. Dabei wird unter einem Single-Domain-Fragment ein Konstrukt, enthaltend eine einzelne erfindungsgemäße variable Antikörperdomäne verstanden und unter einem Single-Chain-Fragment ein Konstrukt, enthaltend eine erfindungsgemäße schwere und leichte Antikörperkette, welche über einen Linker miteinander verknüpft sind.

Die vorliegende Erfindung betrifft auch humanisierte monoklonale Antikörper (humMAk) oder funktionell aktive Teile davon, welche die erfindungsgemäße leichte und schwere Antikörperkette enthalten. Funktionell aktive Teile davon sind beispielsweise F(ab)- oder F(ab')-Fragmente deren Hinge-Region von einem oder mehreren Hinge Exons kodiert werden.

Ferner können die erfindungsgemäßen molekularen Konstrukte oder die erfindungsgemäßen humMAk Effektormoleküle enthalten, beispielsweise ein Chelat (z. B. EDTA, DTPA) zur Komplexierung von Metallionen, ein Toxin (z.B. Ricin), eine zweite Binderegion anderer Spezifität bzw. mit katalytischen Eigenschaften oder ein Enzym. Als Enzyme sind alle im menschlichen Organismus nicht vorkommenden Enzyme, wie beispielsweise die β-Lactamase oder die Carboxypeptidase bevorzugt. Von den im menschlichen Organismus vorkommenden Enzymen können im allgemeinen die Enzyme verwendet werden, die extrazellulär vorzugweise in geringen Konzentrationen vorkommen, wie beispielsweise alle lysosomalen Glucuronidasen oder alle Sialidasen, vorzugsweise die humane Sialidase, die das Sialyl Lew^{x} Epitop zu Lew^{x} modifiziert. Die Sialidase ist besonders vorteilhaft bei der Behandlung von Erkrankungen, die mit einer veränderten Funktion der Granulozyten einhergehen, wie z.B. der Sepsis. Durch die Desialylierung des Sialyl Lew^{x} Epitops wird die Interaktion von aktivierten Granulozyten mit dem vaskulären Endothel unterbunden und somit die Auswanderung der Granulozyten aus dem Blutgefäßsystem beeinträchtigt. Hierdurch wird der Entzündungsvorgang, welcher extravaskulär abläuft, vermindert oder geblockt. Die erfindungsgemäßen Konstrukte oder die erfindungsgemäßen humMAk können beispielsweise nach der Methode von Schwarz (Schwarz, A. & Steinsträsser, A. (1987), A novel approach to TC-99 labelled monoclonal antibodies. J. Nucl. Med., 28, 721) mit Tc-99m oder gemäß allgemein bekannten Methoden mit Re oder Y markiert werden. Die Erfindung schließt im allgemeinen auch jede beliebige andere Markierung mit Alpha-, Beta- oder Gamma-Strahlern ein.

Die Verknüpfung der erfindungsgemäßen Antikörperketten mit Effektormolekülen kann entweder chemisch oder mit Hilfe gentechnischer Methoden nach allgemein bekannten Verfahren vorgenommen werden. Die erfindungsgemäßen Antikörperketten selbst können ebenso chemisch oder gentechnisch hergestellt werden. In letzterem Fall werden, wenn das molekulare Konstrukt, z. B. ein humMAK, nicht aus einem intakten Antikörpermolekül besteht, sondern aus einem Fab oder F(ab')₂ Fragment, bei der Konstruktion der Gene für die schweren Ketten die mit der Entfernung des konstanten Teils des Antigens verloren gegangenen 3' Regulationssequenzen (Stop Codon, 3' nicht-translatierter Bereich und poly-A Additionssignal) durch das C3 Exon, den 3' nicht-translatierten Bereich und das poly-A Additionssignal eines MHC Klasse I Gens, vorzugsweise eines menschlichen HLA B27 Gens, ersetzt.

Generell kann die Wirtszelle im Rahmen der gentechnischen Herstellung der erfindungsgemäßen Antikörperketten bzw. der molekularen Konstrukte mit einem oder mehreren Vektor(en) transfiziert werden. Ein Vektor enthält beispielsweise ein Operon, das für ein von einer leichten Antikörperkette abgeleitetes Polypeptid kodiert und ein zweiter Vektor enthält beispielsweise ein Operon, das für ein von einer schweren Antikörperkette abgeleitetes Polypeptid kodiert. Vorzugsweise sind in diesem Beispiel die beiden Vektoren mit Ausnahme der für die leichten und schweren Ketten kodierenden Bereiche identisch, um eine möglichst gleich gute Expression der leichten und schweren Ketten zu gewährleisten. Weitere Vektoren enthalten im allgemeinen selektierbare Marker.

Alternativ kann für die Transfektion der Wirtszelle z. B. auch ein Vektor verwendet werden, welcher sowohl die für die leichte Kette kodierenden als auch die für die schwere Kette kodierenden DNA-Sequenzen enthält.

Die DNA Sequenzen, welche für die leichten und schweren Ketten kodieren, bestehen entweder aus genomischen Sequenzen oder aus cDNA Sequenzen oder aus einer Mischung von beiden. Vorzugsweise bestehen die genannten DNA Sequenzen zumindest teilweise aus genomischer DNA.

Die Wirtszelle, welche für die Expression der erfindungsgemäßen Antikörperkette(n) bzw. der erfindungsgemäßen molekularen Konstrukte verwendet wird, ist bevorzugt eine eukaryotische Zelle, im besonderen eine Säugerzelle, wie zum Beispiel eine BHK-Zelle, eine CHO-Zelle oder eine myeloide Zelle.

Weitere Gegenstände der vorliegenden Erfindung sind daher auch Klonierungs- und Expressionsvektoren und transfizierte Zellen. Zudem umfaßt die Erfindung therapeutische und diagnostische Formulierungen, welche die erfindungsgemäßen Konstrukte enthalten und deren Herstellung sowie die Verwendung solcher Formulierungen in der Therapie und Diagnose von Erkrankungen, vorzugsweise Entzündungen oder in das Knochenmark metastasierenden Tumoren, insbesondere Mammakarzinom, Lymphom, Prostatakarzinom oder kleinzelligem Lungenkarzinom.

Die generellen Methoden, mit denen die Vektoren konstruiert werden können, die Transfektionstechnologie und die Zellkulturtechnologie sind dem Fachmann bekannt und zum Beispiel von Maniatis (Sambrook, Fritsch, Maniatis; Molecular Cloning, A Laboratory Manual; Cold Spring Harbor Laboratory, S. 11-44, 51-127, 133-134, 141, 146, 150-167, 170, 188-193, 197-199, 248-255, 270-294, 310-328, 364-401, 437-506 (1982); Sambrook, Fritsch, Maniatis; Molecular Cloning, A Laboratory Manual, Second Edition; Cold Spring Harbor Press, S. 16.2-16.22, 16.30-16.40, 16.54-16.55 (1989) beschrieben.

Die vorliegende Erfindung wird nun anhand von Beispielen und durch die Ansprüche näher beschrieben.

### Beispiel 1:

Die V-Gen Sequenzen des Maus MAk BW 250/183 sind in EP-A1-388 914 beschrieben (MAk A):

VH

CAG GTC CAA CTG CAG GAG TCT GGA GGA GGC TTG GTA CAG CCT GGG
Gln Val Gln Leu Gln Glu Ser Gly Gly Gly Leu Val Gln Pro Gly
                5                   10                  15

GGT TCT CTG AGA CTC TCC TGC GCA ACT TCT GGG TTC AGT GAT TAC
Gly Ser Leu Arg Leu Ser Cys Ala Thr Ser Gly Phe Ser Asp Tyr
                20                  25          30

TAC ATG AAC TGG GTC CGC CAG CCT CCA GGA AAA GCA CTT GAG TGG
Tyr Met Asn Trp Val Arg Gln Pro Pro Gly Lys Ala Leu Glu Trp
        35                  40                  45

TTG GGT TTT ATT TCA AAC AAA CCT AAT GGT CAC ACA ACA GAG TAC
Leu Gly Phe Ile Ser Asn Lys Pro Asn Gly His Thr Thr Glu Tyr
        50                              55

AGT GCA TCT GTG AAG GGT CGG TTC ACC ATC TCC AGA GAT AAT TCC
Ser Ala Ser Val Lys Gly Arg Phe Thr Ile Ser Arg Asp Asn Ser
 60                 65                  70

CAA AGC ATC CTC TAT CTT CAA ATG AAC ACC CTG AGA GCT GAG GAC
Gln Ser Ile Leu Tyr Leu Gln Met Asn Thr Leu Arg Ala Glu Asp
 75                 80                              85

AGT GCC ACT TAT TAT TGT GCA AGA GAT AAG GGA ATA CGA TGG TAC
Ser Ala Thr Tyr Tyr Cys Ala Arg Asp Lys Gly Ile Arg Trp Tyr
            90                  95                  100

TTC GAT GTC TGG GGC CAA GGG ACC ACG GTC ACC GTC TCC TCA
Phe Asp Val Trp Gly Gln Gly Thr Thr Val Thr Val Ser Ser
105 110

Die Aminosäurepositionen sind nach Wu und Kabat, "Sequences of Proteins of Immunological Interest", U.S. Department of Health and Human Services", numeriert.

VK

GAC ATT CAG CTG ACC CAG TCT CCA GCA ATC CTG TCT GCA TCT CCA
Asp Ile Gln Leu Thr Gln Ser Pro Ala Ile Leu Ser Ala Ser Pro
                5                   10                  15

GGG GAG AAG GTC ACA ATG ACT TGC AGG GCC AGC TCA AGT GTA AGT
Gly Glu Lys Val Thr Met Thr Cys Arg Ala Ser Ser Ser Val Ser
20 25

TAC ATG CAC TGG TAC CAG CAG AAG CCA GGA TCC TCC CCC AAA CCC
Tyr Met His Trp Tyr Gln Gln Lys Pro Gly Ser Ser Pro Lys Pro
                35                  40                  45

TGG ATT TAT GCC ACA TCC AAC CTG GCT TCT GGA GTC CCT GCT CGC
Trp Ile Tyr Ala Thr Ser Asn Leu Ala Ser Gly Val Pro Ala Arg
                50                  55                  60

TTC AGT GGC AGT GGG TCT GGG ACC TCT TAC TCT CTC ACA ATC ATC
Phe Ser Gly Ser Gly Ser Gly Thr Ser Tyr Ser Leu Thr Ile Ile
                65                  70                  75

AGA GTG GAG GCT GAA GAT GCT GCC ACT TAT TAC TGC CAG CAG TGG
Arg Val Trp Ala Glu Asp Ala Ala Thr Tyr Tyr Cys Gln Gln Trp
                80                  85                  90

AGT AGT AAC CCG CTC ACG TTC GGT GCT GGG ACC AAG CTG GAG ATC
Ser Ser Asn Pro Leu Thr Phe Gly Ala Gly Thr Lys Leu Glu Ile
                95                  100                 105

Als menschliche Empfänger V-Domänen wurden die Myelomproteine NEW (V_{H}) und REI (V_{L}) verwendet (Poljak, R.J., Amzel, L.M., Chen, B.L., Phizackerley, R.P., Saul, F. (1974) Proc. Natl. Acad. Sci. USA 71, 3440-3444; Palm, W., Hilschmann, N. (1973) Z. Physiol. Chem. 354, 1651-1654; Palm, W., Hilschmann, N. (1975) Z. Physiol. Chem. 356, 167-191). Beim Entwurf der Oligonukleotide für die Transplantation der CDR Regionen des MAk BW 250/183 auf die menschlichen Empfänger V-Domänen wurde, wie bei Riechmann, L., Michael Clark, Herman Waldmann, and Greg Winter beschrieben (Nature 332, 323-327, 1988), verfahren:

Die Humanisierung wurde nach der von L. Riechmann et al. und Güssow und Seemann (Methods in Enzymology, 203, 99-121, 1991) beschriebenen Methode vorgenommen, wobei bei der schweren Kette zusätzlich zu den CDR Bereichen (Tramontano, A:, Chothia, C., Lesk, A.M. (1990) J. Mol. Biol. 215, 175-182) die Aminosäureposition 27 in NEW von Ser nach Phe verändert, die Aminosäurepositionen 28 NEW und 29 NEW deletiert und die Position 71 NEW von Val nach Arg verändert wurde.

Daraus resultieren folgende Oligonukleotide für die spezifische Mutagenese:

250/183 Vk CDR1:

5' CTG CTG GTA CCA GTG CAT GTA ACT TAC ACT TGA GCT
GGC CCT ACA GGT GAT GGT 3'

250/183 Vk CDR2:

5' GCT TGG CAC ACC AGA AGC CAG GTT GGA TGT GGC GTA
GAT CAG CAG 3'

250/183 Vk CDR3:

5' CCC TTG GCC GAA CGT GAG CGG GTT ACT ACT CCA CTG
CTG GCA GTA GTA GGT 3'

250/183 VH CDR1:

5' CTG TCT CAC CCA GTT CAT GTA GTA ATC GCT GAA GCC
AGA CAC GTT 3'

250/183 VH CDR2:

5' CTT GCT GGT GTC TCT GAG CAT TGT CAC TCT ACC CTT
CAC GGA TGC GCT GTA CTC GGT TGT GTG TCC GTT AGG
CTT GTT TGA AAT AAA TCC AAT CCA CTC 3'

250/183 VH CDR3:

5' GCC TGG ACC CCA GAC ATC GAA GTA CCA TCG TAT TCC
CTT ATC TCT TGC ACA ATA 3'

Für die Mutagenesen wurden die V_{H} und V_{K} Gene des humanisierten anti Lysozym Antikörpers D1-3 (Verhoeyen, M., Michael, Clark, Herman Waldmann, Greg Winter (1988), Nature 332, 323-327) verwendet. Daraus resultierten die in Tab. la und 1b dargestellten Sequenzen der ersten humanisierten Versionen der V_{H} und V_{K} Gene des MAk 250/183.

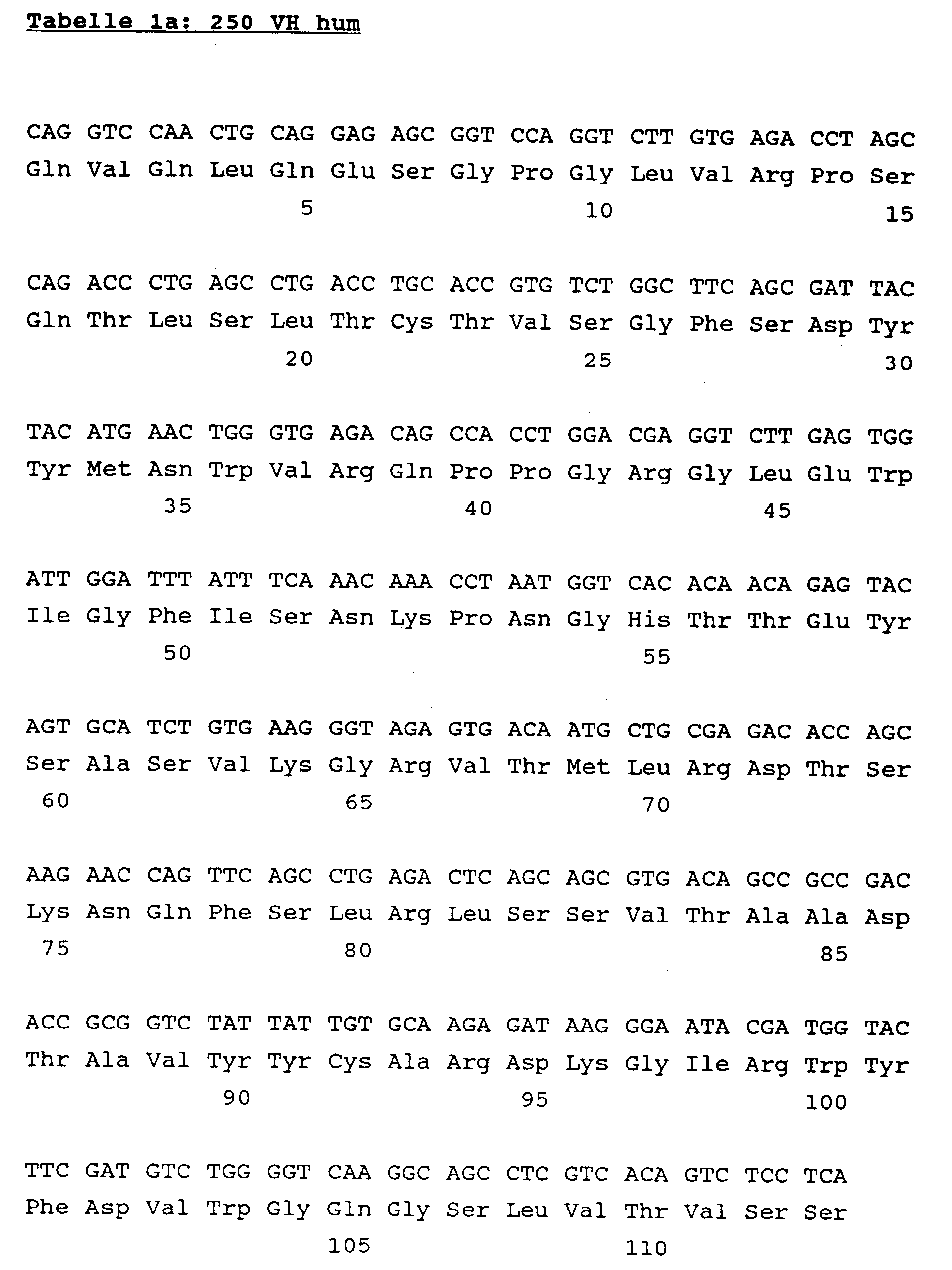

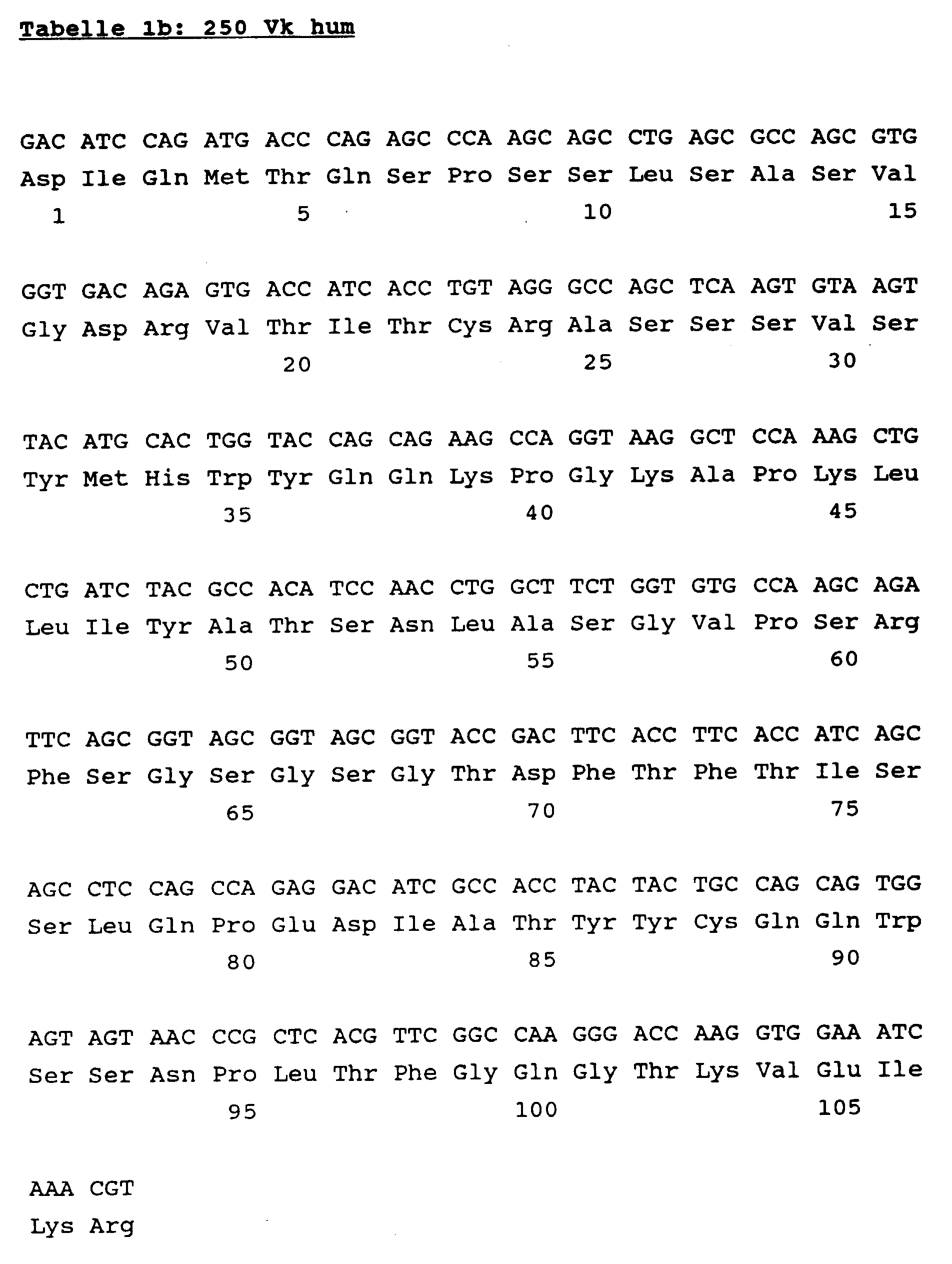

Die humanisierten V_{H} und V_{L} Gene wurden in Expressionsvektoren kloniert, welche das humane IgG3-Gen (DE-A1-3825615) und das humane C_{K}-Gen (Hieter, p.A., Maizel, J.V., Leder, P. (1982), J. Biol. Chem. 257, 1516-1522) enthalten und zusammen mit geeigneten, selektionsmarkertragenden Plasmiden wie z.B. pRMH140 (Hudziak, R.M., Laski, F.A., RajBhandary, U.L., Sharp, P.A., Capecchi, M.R. (1982) Cell 31, 137-146) oder pSV2 dhfr (Lee, F., Mulligan, R., Berg, P., Ringold, G. (1981) Nature 294, 228) in Säugerzellen transfiziert (Wirth, M., Bode, J., Zettlmeißl, G., Hauser, H. (1988) Gene 73, 419-426).

Die Charakterisierung dieser ersten Version des humanisierten Antikörpers (gemäß Beispiel 2) zeigte jedoch eine im Vergleich zum Maus MAk stark reduzierte Affinität zum Antigen.

Bei einer erneuten Mutagenese der Aminosäuresequenzen wurde bei der V_{L} Domäne zusätzlich zu den CDRs die Aminosäureposition REI 46 von Leu in Pro verändert. Daraus resultierte folgendes Oligonukleotid (250/183 V_{K} CDR2neu) für die Mutagenese des CDR2 der ersten Version des humanisierten 250/183 V_{K} Gens:

250/183 Vk CDR2 neu:

5' GCT TGG CAC ACC AGA AGC CAG GTT GGA TGT GGC GTA
GAT CAG CGG CTT TGG AGC CTT 3'

Die aus dieser Mutagenese resultierende Sequenz der zweiten Version des humaniserten leichten Ketten V-Gens ist in Tabelle 1c dargestellt.

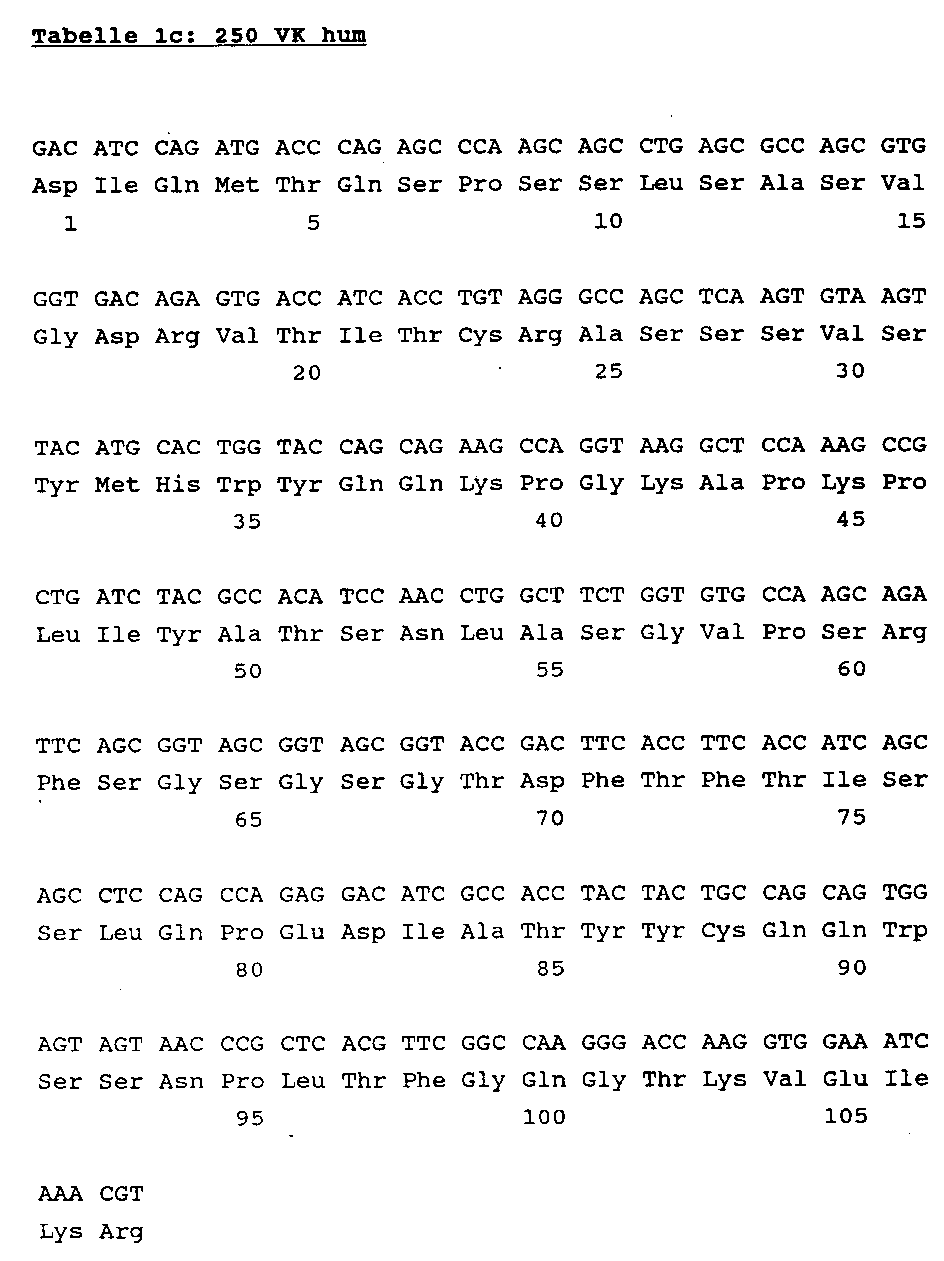

### Beispiel 2:

### Immunologische Prüfung des BW 250/183 hum

Überstände von BHK Zellen, die mit den DNA-Konstrukten der zweiten Version des hum 250/183 transfiziert wurden, wurden auf humMAk Gehalt und Spezifität des humMAk geprüft.

Hierzu wurden 2 verschiedene ELISA-Tests eingesetzt:
a) ELISA zur Bestimmung der humanen IgG Konzentration
b) Epitop-Kompetitions-ELISA auf Festphasenantigen

- zu a): Der ELISA zur Bestimmung der humanen IgG Konzentration wurde entsprechend der bei Johannson et. al. (J. Immunol. Methods 87, 7-11) beschriebenen Methode durchgeführt.

Als Festphasenantikörper wurde ein Ziege-Anti-Human-IgG (Southern Biotechnology Associates (Best. Nr. 2040-01)) 1:300 verdünnt. Als Nachweisantikörper diente ein alkalischer phosphatasemarkierter Ziege-Anti-Human-Kappa-Antikörper von der gleichen Firma (Best.Nr.: 2060-04), der in einer Verdünnung von 1:250 eingesetzt wurde.
- zu b): Der Epitop-Kompetitions-ELISA wurde ebenfalls wie bei Johannson et al. (s.o.) beschrieben durchgeführt. Allerdings wurden die Polystyrolplatten mit 75 µl CEA von einer Konzentration von 15 µg/ml gecoatet. Der Transfektomüberstand, der 200 ng/ml hum 250/183 enthielt, wurde mit gleichen Volumen verschiedener Konzentrationen (fortlaufende Verdünnungen 1:2) der murinen Kompetitoren versetzt und 15 Minuten bei Raumtemperatur inkubiert. Anschließend wurde das Gemisch für 20 Stunden bei Raumtemperatur auf die CEA-Festphase gegeben und der CEA-gebundene hum 250/183 mittels eines 1:250 verdünnten, mit alkalischer Phosphatase markierten Ziege- Anti-Human-Kappa-Antikörpers (Best.Nr.: 2060-04) der Firma Southern Biotechnology Associates nachgewiesen.

### Ergebnis:

Mittels des ELISAs zum Nachweis von humanem IgG konnten Überstände von transfizierten BHK-Zellen gefunden werden, die ca. 200 ng/ml humanes IgG enthielten. Diese Überstände wurden dann in einem Epitop-Kompetitions-ELISA auf Spezifität geprüft.

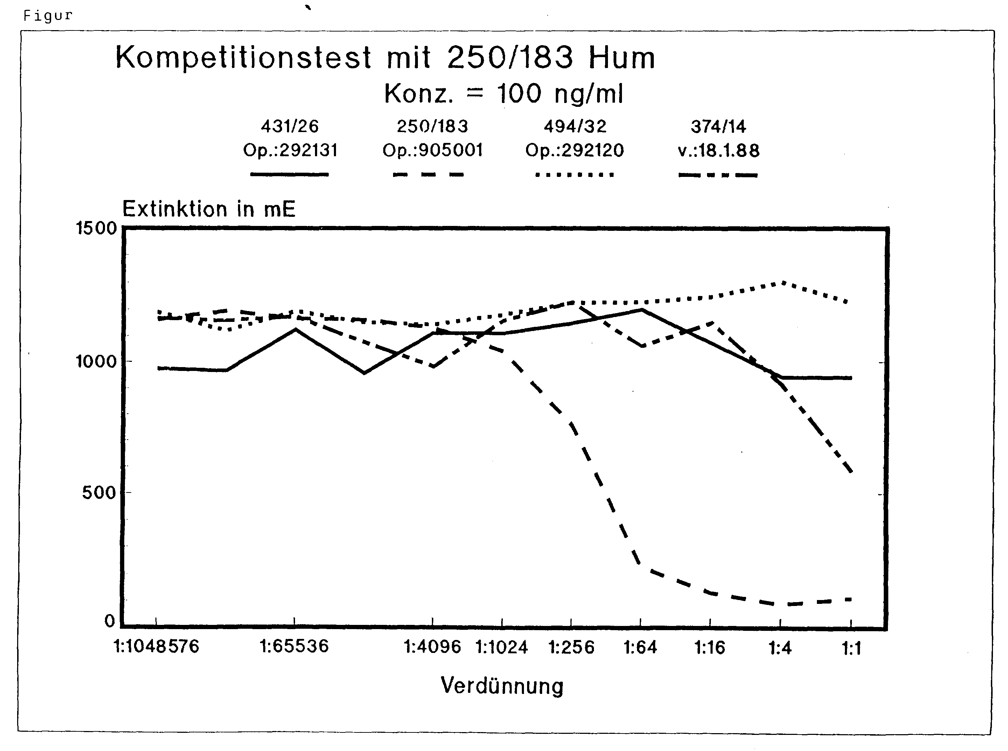

Die Figur zeigt, daß von den zur Kompetition im Überschuß eingesetzten murinen MAk nur der MAk BW 250/183 die Bindung des hum 250/183 an sein CEA-NCA95 spezifisches Epitop, welches auf der Festphase fixiert war, dosisabhängig inhibiert. Die MAk BW 431/26 und BW 374/14, die andere Epitope auf CEA erkennen (Bosslet, K., et al., Int. J. Cancer 36, 75-84, 1985; s.o.) beeinflussen die Bindung des hum 250/183 ebenso wenig wie der TFβ-spezifische MAk BW 494/32 (Bosslet, et al. Cancer Immunol. Immunother. 23, 185-189, 1986). Diese Daten zeigen, daß die Humanisierung des MAk BW 250/183 die Epitopspezifität nicht meßbar verändert hat, d.h. die Spezifität des hum 250/183 ist identisch mit der des Maus MAk BW 250/183.

## Patentansprüche

1. Leichte menschliche granulozytenbindende Antikörperkette, die von einem monoklonalen Mausantikörper abgeleitet wurde, enthaltend ein Polypeptid der Aminosäuresequenz 
Asp Ile Gln Met Thr Gln Ser Pro Ser Ser Leu Ser Ala Ser
Val Gly Asp Arg Val Thr Ile Thr Cys Arg Ala Ser Ser Ser
Val Ser Tyr Met His Trp Tyr Gln Gln Lys Pro Gly Lys Ala
Pro Lys Pro Leu Ile Tyr Ala Thr Ser Asn Leu Ala Ser Gly
Val Pro Ser Arg Phe Ser Gly Ser Gly Ser Gly Thr Asp Phe
Thr Phe Thr Ile Ser Ser Leu Gln Pro Glu Asp Ile Ala Thr
Tyr Tyr Cys Gln Gln Trp Ser Ser Asn Pro Leu Thr Phe Gly
Gln Gly Thr Lys Val Glu Ile Lys Arg
 oder funktionell äquivalente Varianten davon, die an menschliche Granulozyten oder Granulozytenvorläufer, an NCA 95 und CEA binden, jedoch nicht an menschlichen Normalgewebe außer der Colonmucosa, wobei die Aminosäure in Position 45 (Pro) nicht gegen eine andere Aminosäure ausgetauscht werden darf.

2. Antikörper, enthaltend mindestens eine leichte menschliche granulozytenbindende Antikörperkette gemäß Anspruch 1 und mindestens eine schwere Antikörperkette , enthaltend ein Polypeptid der Aminosäuresequenz 
Gln Val Gln Leu Gln Glu Ser Gly Pro Gly Leu Val Arg Pro
Ser Gln Thr Leu Ser Leu Thr Cys Thr Val Ser Gly Phe Ser
Asp Tyr Tyr Met Asn Trp Val Arg Gln Pro Pro Gly Arg Gly
Leu Glu Trp Ile Gly Phe Ile Ser Asn Lys Pro Asn Gly His
Thr Thr Glu Tyr Ser Ala Ser Val Lys Gly Arg Val Thr Met
Leu Arg Asp Thr Ser Lys Asn Gln Phe Ser Leu Arg Leu Ser
Ser Val Thr Ala Ala Asp Thr Ala Val Tyr Tyr Cys Ala Arg
 
Asp Lys Gly Ile Arg Trp Tyr Phe Asp Val Trp Gly Gln Gly
Ser Leu Val Thr Val Ser Ser
 oder funktionell äquivalente Varianten davon, die an menschliche Granulozyten oder Granulozytenvorläufer, an NCA 95 und CEA binden, jedoch nicht an menschlichen Normalgewebe außer der Colonmucosa.

3. Molekulares Konstrukt enthaltend mindestens eine Antikörperkette nach Anspruch 1 oder 2 und mindestens eine weitere mit der Antikörperkette verknüpfte Aminosäurekette.

4. Molekulares Konstrukt nach Anspruch 3, dadurch gekennzeichnet, daß es ein Single-Chain-Fragment ist.

5. Molekulares Konstrukt nach Anspruch 3, dadurch gekennzeichnet, daß es ein Single-Domain-Fragment ist.

6. Molekulares Konstrukt nach Anspruch 3, dadurch gekennzeichnet, daß die weiteren Aminosäureketten Teile eines humanen Antikörpers und verschieden von den Sequenzen gemäß Tabelle 1a oder 1c sind.

7. Molekulares Konstrukt nach Anspruch 6, dadurch gekennzeichnet, daß es ein humanisierter Antikörper ist.

8. Humanisierter Antikörper nach Anspruch 2 oder ein funktionell aktiver Teil davon.

9. Humanisierter Antikörper nach Anspruch 8, dadurch gekennzeichnet, daß der funktionell aktive Teil ein F(ab)- oder ein F(ab')-Fragment, dessen Hinge-Region von einem oder mehreren Hinge exons kodiert wird, ist.

10. Molekulares Konstrukt nach Anspruch 3, dadurch gekennzeichnet, daß es mit einem Effektormolekül verbunden ist, wobei das Effektormolekül aus der Gruppe Chelat zur Komplexierung von Metallionen, wie EDTA oder DTPA, Toxin wie Ricin, eine andere Binderegion anderer Spezifität oder mit katalytischen Eigenschaften oder Enzym ausgewählt wird.

11. Polynukleotid, das für die Antikörperkette nach Anspruch 1 kodiert.

12. Polynukleotid, das für die Antikörperkette nach Anspruch 2 kodiert.

13. Verfahren zur Herstellung eines molekularen Konstruktes nach Anspruch 3, dadurch gekennzeichnet, daß
a) in einem Expressionsvektor ein Operon hergestellt wird, das mindestens ein Polynukleotid nach Anspruch 11 oder 12 enthält;
b) der genannte Expressionsvektor in eine Wirtszelle eingebracht wird und
c) die Wirtszelle kultiviert und
d) das molekulare Konstrukt isoliert wird.

14. Verfahren nach Anspruch 13, dadurch gekennzeichnet, daß ein Operon hergestellt wird, bei dessen Konstruktion der für die konstante Region des Immunglobulins kodierende Bereich deletiert und die dadurch verlorengegangene 3'-Regulationssequenz durch das C3-Exon, den 3' nicht translatierten Bereich und das poly-A Additionssignal eines MHC Klasse 1-Gens ersetzt wird.

15. Molekulares Konstrukt nach Anspruch 3 zum Nachweis von Entzündungen und/oder zum Nachweis von in das Knochenmark metastasierenden Tumoren, vorzugsweise Mammakarzinom, Lymphom, Prostatakarzinom oder kleinzelliges Lungenkarzinom.

16. Verwendung des molekularen Konstruktes nach Anspruch 3 zur Herstellung eines Arzneimittels gegen Entzündungen und in das Knochenmark metastasierende Tumore, vorzugsweise Mammakarzinom, Lymphom, Prostatakarzinom oder kleinzelliges Lungenkarzinom.

17. Diagnostikum, enthaltend ein molekulares Konstrukt nach Anspruch 3.

18. Arzneimittel, enthaltend ein molekulares Konstrukt nach Anspruch 3.

## Claims

1. A light human granulocyte-binding antibody chain which was derived from a monoclonal mouse antibody, containing a polypeptide of the amino acid sequence 
Asp Ile Gln Met Thr Gln Ser Pro Ser Ser Leu Ser Ala
Ser Val Gly Asp Arg Val Thr Ile Thr Cys Arg Ala Ser
Ser Ser Val Ser Tyr Met His Trp Tyr Gln Gln Lys Pro
Gly Lys Ala Pro Lys Pro Leu Ile Tyr Ala Thr Ser Asn
Leu Ala Ser Gly Val Pro Ser Arg Phe Ser Gly Ser Gly
Ser Gly Thr Asp Phe Thr Phe Thr Ile Ser Ser Leu Gln
Pro Glu Asp Ile Ala Thr Tyr Tyr Cys Gln Gln Trp Ser
Ser Asn Pro Leu Thr Phe Gly Gln Gly Thr Lys Val Glu
Ile Lys Arg
 or functionally equivalent variants thereof which bind to human granulocytes or granulocyte precursors, to NCA 95 and CEA, but not to normal human tissue apart from the colon mucosa, it not being permissible for the amino acid in position 45 (Pro) to be exchanged for another amino acid.

2. An antibody, containing at least one light human granulocyte-binding antibody chain as claimed in claim 1 and at least one heavy antibody chain, containing a polypeptide of the amino acid sequence 
Gln Val Gln Leu Gln Glu Ser Gly Pro Gly Leu Val Arg
Pro Ser Gln Thr Leu Ser Leu Thr Cys Thr Val Ser Gly
Phe Ser Asp Tyr Tyr Met Asn Trp Val Arg Gln Pro Pro
Gly Arg Gly Leu Glu Trp Ile Gly Phe Ile Ser Asn Lys
Pro Asn Gly His Thr Thr Glu Tyr Ser Ala Ser Val Lys
Gly Arg Val Thr Met Leu Arg Asp Thr Ser Lys Asn Gln
Phe Ser Leu Arg Leu Ser Ser Val Thr Ala Ala Asp Thr
Ala Val Tyr Tyr Cys Ala Arg Asp Lys Gly Ile Arg Trp
Tyr Phe Asp Val Trp Gly Gln Gly Ser Leu Val Thr Val
Ser Ser
 or functionally equivalent variants thereof which bind to human granulocytes or granulocyte precursors, to NCA 95 and CEA but not to normal human tissue apart from the colon mucosa.

3. A molecular construct containing at least one antibody chain as claimed in claim 1 or 2 and at least one further amino acid chain linked to the antibody chain.

4. The molecular construct as claimed in claim 3, wherein it is a single chain fragment.

5. The molecular construct as claimed in claim 3, wherein it is a single domain fragment.

6. The molecular construct as claimed in claim 3, wherein the further amino acid chains are parts of a human antibody and are different from the sequences according to Table 1a or 1c.

7. The molecular construct as claimed in claim 6, wherein it is a humanized antibody.

8. The humanized antibody as claimed in claim 2 or a functionally active part thereof.

9. The humanized antibody as claimed in claim 8, wherein the functionally active part is an F(ab) or an F(ab') fragment whose hinge region is encoded by one or more hinge exons.

10. The molecular construct as claimed in claim 3, wherein it is bound to an effector molecule, the effector molecule being selected from the group consisting of chelate for complexing metal ions, such as EDTA or DTPA, toxin such as ricin, a different binding region of a different specificity or having catalytic properties or enzyme.

11. A polynucleotide which encodes the antibody chain as claimed in claim 1.

12. A polynucleotide which encodes the antibody chain as claimed in claim 2.

13. A process for preparing a molecular construct as claimed in claim 3, wherein
a) an operon is prepared in an expression vector, which operon contains at least one polynucleotide as claimed in claim 11 or 12;
b) the said expression vector is introduced into a host cell and
c) the host cell is cultured and
d) the molecular construct is isolated.

14. The process as claimed in claim 13, wherein an operon is prepared during whose construction the region encoding the constant region of the immunoglobulin is deleted and the 3' regulatory sequence which is thereby lost is replaced by the C3 exon, the 3' nontranslated region and the polyA addition signal of an MHC Class I gene.

15. The molecular construct as claimed in claim 3 for detecting inflammations and/or for detecting tumors which are metastasizing into the bone marrow, preferably carcinoma of the breast, lymphoma, carcinoma of the prostate, or small-cell lung carcinoma.

16. Use of the molecular construct as claimed in claim 3 for preparing a medicament against inflammations and tumors which are metastasizing into the bone marrow, preferably carcinoma of the breast, lymphoma, carcinoma of the prostate, or small-cell lung carcinoma.

17. A diagnostic agent containing a molecular construct as claimed in claim 3.

18. A medicament containing a molecular construct as claimed in claim 3.

## Revendications

1. Chaîne légère d'anticorps humain se liant aux granulocytes, qui a été dérivée d'un anticorps monoclonal de souris, contenant un polypeptide ayant la séquence d'aminoacides: 
Asp Ile Gln Met Thr Gln Ser Pro Ser Ser Leu Ser Ala Ser
Val Gly Asp Arg Val Thr Ile Thr Cys Arg Ala Ser Ser Ser
Val Ser Tyr Met His Trp Tyr Gln Gln Lys Pro Gly Lys Ala
Pro Lys Pro Leu Ile Tyr Ala Thr Ser Asn Leu Ala Ser Gly
Val Pro Ser Arg Phe Ser Gly Ser Gly Ser Gly Thr Asp Phe
Thr Phe Thr Ile Ser Ser Leu Gln Pro Glu Asp Ile Ala Thr
Tyr Tyr Cys Gln Gln Trp Ser Ser Asn Pro Leu Thr Phe Gly
Gln Gly Thr Lys Val Glu Ile Lys Arg
 ou des variantes fonctionnellement équivalentes de celle-ci, qui se lient aux granulocytes humains ou à des précurseurs de granulocytes, à NCA 95 et CEA, mais non à un tissu normal humain hormis la muqueuse du côlon, l'aminoacide en position 45 (Pro) ne devant pas être remplacé par un autre aminoacide.

2. Anticorps contenant au moins une chaîne légère d'anticorps humain se liant aux granulocytes, selon la revendication 1, et au moins une chaîne lourde d'anticorps, contenant un polypeptide ayant la séquence d'aminoacides: 
Gln Val Gln Leu Gln Glu Ser Gly Pro Gly Leu Val Arg Pro
Ser Gln Thr Leu Ser Leu Thr Cys Thr Val Ser Gly Phe Ser
Asp Tyr Tyr Met Asn Trp Val Arg Gln Pro Pro Gly Arg Gly
Leu Glu Trp Ile Gly Phe Ile Ser Asn Lys Pro Asn Gly His
Thr Thr Glu Tyr Ser Ala Ser Val Lys Gly Arg Val Thr Met
Leu Arg Asp Thr Ser Lys Asn Gln Phe Ser Leu Arg Leu Ser
Ser Val Thr Ala Ala Asp Thr Ala Val Tyr Tyr Cys Ala Arg
Asp Lys Gly Ile Arg Trp Tyr Phe Asp Val Trp Gly Gln Gly
Ser Leu Val Thr Val Ser Ser
 ou des variantes fonctionnellement équivalentes de celle-ci, qui se lient aux granulocytes humains ou à des précurseurs de granulocytes, à NCA 95 et CEA, mais non à un tissu normal humain hormis la muqueuse du côlon.

3. Produit de construction moléculaire, contenant au moins une chaîne d'anticorps selon la revendication 1 ou 2 et au moins une autre chaîne d'aminoacides liée à la chaîne d'anticorps.

4. Produit de construction moléculaire selon la revendication 3, caractérisé en ce qu'il est un fragment d'une seule chaîne.

5. Produit de construction moléculaire selon la revendication 3, caractérisé en ce qu'il est un fragment d'un seul domaine.

6. Produit de construction moléculaire selon la revendication 3, caractérisé en ce que les autres chaînes d'aminoacides sont des parties d'un anticorps humain et sont différentes des séquences selon le tableau 1a ou 1c.

7. Produit de construction moléculaire selon la revendication 6, caractérisé en ce qu'il est un anticorps humanisé.

8. Anticorps humanisé selon la revendication 2 ou partie fonctionnellement active de celui-ci.

9. Anticorps humanisé selon la revendication 8, caractérisé en ce que la partie fonctionnellement active est un fragment F(ab) ou un fragment F(ab'), dont la région charnière est codée par un ou plusieurs exons de charnière.

10. Produit de construction moléculaire selon la revendication 3, caractérisé en ce qu'il est lié à une molécule d'effecteur, la molécule d'effecteur étant choisie dans le groupe constitué par un chélate pour la complexation d'ions métalliques, tels qu'EDTA ou DTPA, une toxine telle que la ricine, une autre région de liaison ayant une autre spécificité ou à propriétés catalytiques, ou une enzyme.

11. Polynucléotide codant pour la chaîne d'anticorps selon la revendication 1.

12. Polynucléotide codant pour la chaîne d'anticorps selon la revendication 2.

13. Procédé pour la production d'un produit de construction moléculaire selon la revendication 3, caractérisé en ce que
a) on produit dans un vecteur d'expression un opéron qui contient au moins un polynucléotide selon la revendication 11 ou 12,
b) on introduit ledit vecteur d'expression dans une cellule hôte et
c) on cultive la cellule hôte et
d) on isole le produit de construction moléculaire.

14. Procédé selon la revendication 13, caractérisé en ce que l'on produit un opéron dans la construction duquel la zone codant pour la région constante de l'immunoglobuline est supprimée et la séquence de régulation en 3' ainsi perdue est remplacée par l'exon C3, la zone non traduite en 3' et le signal d'addition poly-A d'un gène de classe I du CMH.

15. Produit de construction moléculaire selon la revendication 3, pour la détection d'inflammations et/ou pour la détection de tumeurs disséminant des métastases dans la moelle osseuse, de préférence le cancer du sein, un lymphome, le cancer de la prostate ou le cancer du poumon à petites cellules.

16. Utilisation du produit de construction moléculaire selon la revendication 3, pour la fabrication d'un médicament contre des inflammations et des tumeurs disséminant des métastases dans la moelle osseuse, de préférence le cancer du sein, un lymphome, le cancer de la prostate ou le cancer du poumon à petites cellules.

17. Agent de diagnostic, contenant un produit de construction moléculaire selon la revendication 3.

18. Médicament, contenant un produit de construction moléculaire selon la revendication 3.
